# EUROPEAN PATENT SPECIFICATION

(11) **EP 4 031 038 B1**
(45) Date of publication and mention of the grant of the patent: **04.12.2024**
(21) Application number: 20772258.8
(22) Date of filing: 14.09.2020
(51) Int. Cl.: A61B 17/80, A61B 17/17, A61B 17/86

(54) **BEAD HEAD FOR LOCKING BONE SCREWS**
WULSTKOPF ZUM VERRIEGELN VON KNOCHENSCHRAUBEN
TÊTE DE BILLE POUR LE VERROUILLAGE DE VIS À OS

(30) Priority: 18.09.2019 EP 19198069
(43) Date of publication of application: 27.07.2022
(73) Proprietor: Kyon AG, 8005 Zürich (CH)
(72) Inventor: TEPIC, Slobodan, 8057 Zurich (CH)
(74) Representative: Weickmann & Weickmann PartmbB
(86) International application number: PCT/EP2020/075617
(87) International publication number: WO 2021/052902

(56) References cited:
- US-A- 5 151 103
- US-A1- 2009 118 768
- US-A1- 2009 248 087
- US-B1- 6 309 393

## Description

### Technical field

The present invention relates to an implant construct comprising a bone locking plate, a bone locking screw and a spherical bead, wherein the bead includes a conical hole and is to be interposed between a conical head of the bone locking screw and a receiving hole in the bone locking plate.

### Background

The defining feature of the present invention is the slightly conical or tapered head of the screws. Commonly referred to as "Morse taper", this mechanical solution for a precise, reliable and reversible coupling of mechanical components invented by Samuel Colt (of Colt handgun fame) in 1854, was adopted by Stephen Ambrose Morse along with an equally lucrative invention of a spiral drill, which he contributed to, leading to formation of Morse Twist Drill and Machine Co. in New Bedford, MA, in 1864, one of the uniquely American industrial manufacturing success stories of the 19^{th}century, surviving to this day.

The original and still most commonly used Morse taper in machinery is defined as 1:20 (e.g. 1 mm change of diameter over a 20 mm axial length), which corresponds to the total angle of the taper of 2.86 degrees. Adopted for use in orthopedic devices for coupling of e.g. heads to the stems in modular hip prosthesis (P. Hernigou, S. Queinnec, C. H. F. Lachaniette: One hundred and fifty years of history of the Morse taper: from Stephen A. Morse in 1864 to complications related to modularity in hip arthroplasty, International Orthopaedics (SICOT) (2013) 37:2081-2088), the taper has been changed to 1:10 (5.72 degrees) with many variations in geometry and fit. The important, unifying feature to all Morse-type tapers is the self-retaining or self-locking character of the connection - once the parts are put together, they will not come apart unless a significant force is used to separate them. The smaller the angle of the taper is, the tighter is the connection. However, the smaller the angle is, the tighter the manufacturing tolerances required are. The ultimate decision is a compromise of the requirements, the risks of separation vs. jamming, the expected coefficient of friction in use, and the manufacturing costs.

The first known, documented use of the Morse taper locking screws for internal fixation plates was in the research and development project carried out at the AO Research Institute, Davos, Switzerland in 1986, under a code name FIXIN (from FIXator INtern), led by the present inventor (see e.g. USP 5,151,103, Tepic et al.). The tapered heads of screws were locking in the plate via interposed, slotted and thus expandable balls. These plates and screws were applied to intact bones only in experimental sheep to study the impact on the periosteal blood supply. Whether the design was sufficiently robust to stabilize fractured bones was not tested because the first findings from the project on intact bones have demonstrated the damage to endosteal blood perfusion caused by conventional use of bi-cortical screws. Decision to switch to use of exclusively mono-cortical screws made the expanding balls un-necessary and the project took on a new name (PC-Fix for Point Contact Fixator), and with new élan at the Davos Institute it proceeded to demonstrate over the next decade with extensive pre-clinical and clinical testing the numerous advantages of keeping blood perfusion of fractured bone as intact as possible (The biomechanics of the PC-Fix internal fixator, Tepic, S. et al., Injury, Volume 26, B5 - B10). The locking of the screw heads in the plates was necessary but preserving the blood perfusion by the periosteum called for further features of the plate design mostly of the bone-facing side of the plates. At that time reduction of the risks of screw heads jamming in the plate were addressed by the unique geometry of the coupling. The Morse taper couplings used in machines are manufactured from solid, hardened stock, ground to high precision and smooth surface finish. Screws and plates of PC-Fix were machined from relatively soft c.p. titanium with a recess for the screwdriver in the head. Preventing jamming due to so-called cold welding of the screw heads in the plates called for some careful observation, design, including Finite Element analysis. The residual risks due to over-tightening of the screws were deemed acceptable including by the surgeons participating in the clinical study in close to 2,000 human patients with forearm fractures. The results were published when the number of enrolled patients with follow-up reached about 1'200, Eijer H, Hauke C, Arens S, Printzen G, Schlegel U, Perren SM. PC-Fix and local infection resistance-influence of implant design on postoperative infection development, clinical and experimental results. Injury. 2001 Sep;32 Suppl 2: B38-43).

However, the three commercial partners of the AO Foundation, later all merged into Synthes, subsequently acquired by Johnson and Johnson, now a part of DePuy-Synthes, did not commercialize PC-Fix and no other company has brought all of its crucial features to clinical use until Kyon's Advanced Locking Plate System (ALPS^{®}) was released for veterinary use in 2007 (USP 8,968,368, Tepic).

The locking mechanism of ALPS^{®} differs from that used in PC-Fix. The screw heads are conical but with a much larger angle and thus not self-retaining. A similar design is used in car wheel lug bolts - the screw threads of ALPS^{®} screws do engage with the (partial) threads in the plate holes as they are threaded into the bone. This design provided new opportunities - each plate hole could accept a downsized regular screw with the same angulation freedom as in the original DCP (Dynamic Compression Plate, of AO/Synthes) plates as well as allowing for the fracture compression function of DCP.

During the years of patent protection for PC-Fix (with the patent rights assigned by the AO Foundation to its commercial partners) the only use of Morse taper screw heads for locking implants was by Kyon in its "Zurich Cementless" THR (USP 5,458,654, Tepic) and by Intrauma, Rivoli TO, Italy for its FIXIN^{™} plating system. The Intrauma locking system relies on screws similar to PC-Fix screws but with a machine-threaded ring interposed between the plate and the screw head. These screw heads invariably jam into the rings, but the rings can be removed from the plate if needed.

An implant construct is further known from the US 2009/248087 A1, from which the preamble of claim 1 derives.

### Summary of the Invention

The present invention is defined in claim 1 while preferred embodiments are set forth in the dependent claims.

The present invention discloses a solution for angulation of bone screws with conical self-locking heads in the bone plates with conical holes. Angulation is made possible with the addition of a spherical bead head interposed between the screw head and the plate hole. To allow insertion of the screw at an angle the hole in the bone is drilled in the bone with aid of a special drill sleeve. Then a bead having a conical hole is inserted into the plate hole with a proper orientation e.g. with aid of a bead-holder. Finally, the screw, down-sized compared to the nominal locking screw, is inserted through the hole of the bead into the bone. That locks the head of the angulated screw inside the bead and the bead inside the plate hole. Furthermore, locking screws can be augmented by the beads to allow them to be used as dynamic compression screws.

The present invention relates to an implant construct comprising a spherical bead with a conical hole for interposition between a locking bone screw head and a receiving hole in a bone plate. The bead may be used for bone fixation in medicinal applications in combination with a fitting bone screw and a bone plate.

In certain embodiments, the conical hole of the bead is tapered with a self-locking angle which may be between about 2.7° and about 8°, particularly between about 4° to about 6.5° and more particularly about 5.7°, corresponding to a screw-taper of 1:10.

The implant construct further comprises a locking screw, and a bone plate. The bead comprises a conical hole and the bone screw is adapted for insertion into a bone through the conical hole of the bead. The screw may comprise a self-locking head for fixation. The bone plate comprises at least one conical receiving hole adapted for insertion of the bead.

In certain embodiments, the screw comprises a conical head wherein the total angle of the conical screw head is larger than the angle of the hole in the bead, particularly by about 0.2° to about 0.4°, more particularly by about 0.3°.

In certain embodiments, the total angle of the conical hole in the bone plate is about the same as the total angle of the conical hole in the spherical bead.

The bead, the bone screw and the bone plate of the present invention can be made of any suitable material, e.g. of a metal or metal alloy. In particular embodiments, the bead, the bone screw and/or the bone plate are made of titanium or a titanium-containing alloy.

There is ample evidence that titanium and its alloys can provide all of the mechanical requirements needed but with improved biocompatibility in comparison to stainless steels, for example, most commonly used 316L, or EN 1.4404.

The human orthopedic industry, however, has been and remains reluctant to abandon its reliance on stainless steel, especially in trauma devices. Thus, in certain embodiments, the bead, the bone screw and/or the bone plate are made of stainless steel.

The bone plates of the present invention may have outside shapes almost identical to those of the original ALPS^{®}, except for the screw holes. Minor adjustments were made to take advantage of now smaller holes to increase the overall strength. The first choice of material for plates is c.p. titanium Grade 4 but there is also an option of titanium alloy (Ti6Al4V), so-called Titanium Grade 5, for plates that may need extra strength. Titanium Grade 4 is slightly weaker than stainless steel 316L most commonly used for plates and screws. However, the shape and size of the implants can be easily adjusted to make up for that difference. For example, plates size 10 of the original ALPS^{®} of the same outside dimensions as the benchmark plate used for comparison, DCP 3.5 from Synthes made in stainless steel 316L, has 20% higher strength in bending. In typical conditions of heat treatment and cold-work used for implants, titanium Grade 5 has about 50% higher strength than Grade 4 or stainless steel 316L. The downside of using Grade 5 is in its lower ductility so the plates cannot be bent as much as those made in Grade 4 or in stainless steel.

The bone screws of the current invention are made preferably from a titanium-aluminum-niobium alloy (Ti6Al7Nb or TAN). Decades after its invention, TAN is getting some attention in the industry. The mechanical properties are identical to those of TAV (Ti6Al4V) but due to replacement of the highly toxic vanadium by the very inert niobium, TAN is about as biocompatible as the pure titanium. Bone adhesion to TAN is superb to the point that removal of integrated TAN implants might be more difficult than of any other metallic implants.

The plates of this invention are preferably treated by micro peening process for increased fatigue strength.

The locking screws with a Morse taper type head, such as in PC-Fix, can be used in almost all circumstances alone in their locking configuration at 90 degrees to the plate, but there is an occasional need for using screws angulated with respect to the plate as well as applying compression across the fracture or osteotomy plane.

To provide for screw angulations of the same amplitude as in the original ALPS^{®}, which is the same as in DCP, a new solution, disclosed herein, was found by the addition of beads. The beads may be of spherical shape outside and are provided with a conical hole to receive the locking head of the screws. The utility of the beads goes past the obvious conversion of a conical to a spherical head. If the screws with spherical heads were to be inserted into the conical holes of the plates, the plates could not be held in their axial position on the bone - the screws with spherical heads (or augmented with bead heads) inserted at an angle would hit the top of the receiving hole before the head could be seated in the plate hole. If such screws were used only one per plate, this axial slip could be tolerated but that angulated screw would have to be inserted as the first screw, which is generally not an acceptable restriction to fixing a plate to a fractured bone. Drilling of the hole for the angulated screw is possible with a dedicated drill sleeve. The bead is then inserted into the screw hole in the plate with aid of a bead holder. Once the bead is firmly seated in the plate hole, a locking screw, e.g. downsized from the regular screw, can be screwed through the bead into the bone until it safely locks in the bead. The bead is then also locked in the plate, albeit not as strongly as a conical head screw. Use of the beads in conjunction with locking screws is indicated only seldom, e.g. for screws very close to joints or possibly for use as compression screws. It should be noted that the dynamic compression principle cannot be used unless the fracture (osteotomy) is close to transverse. In veterinary (but also in human) trauma surgery a great majority of fractures are oblique, spiral or comminuted and thus not amenable to treatment by interfragmentary compression.

### List of figures:

1. Locking bone screw with Morse taper-type head.
2. Transverse and longitudinal cross-sections of the bone plate with a conical hole.
3. Perspective views of a bone plate section with a conical hole.
4. Locking screw inserted in the plate in transverse and in longitudinal cross-sections.
5. Bead for the conical locking screw head.
6. A bone screw inserted through a bead angulated in the transverse plane.
7. A bone screw inserted through a bead angulated in the longitudinal plane.
8. Drill sleeve seated in the conical hole, angulated in the longitudinal direction before drilling a hole in the bone is performed.
9. Use of the beads to allow for angulation of the bone screws.
10. Offset screw placement for interfragmentary compression using a locking screw augmented with a bead.

### Detailed description

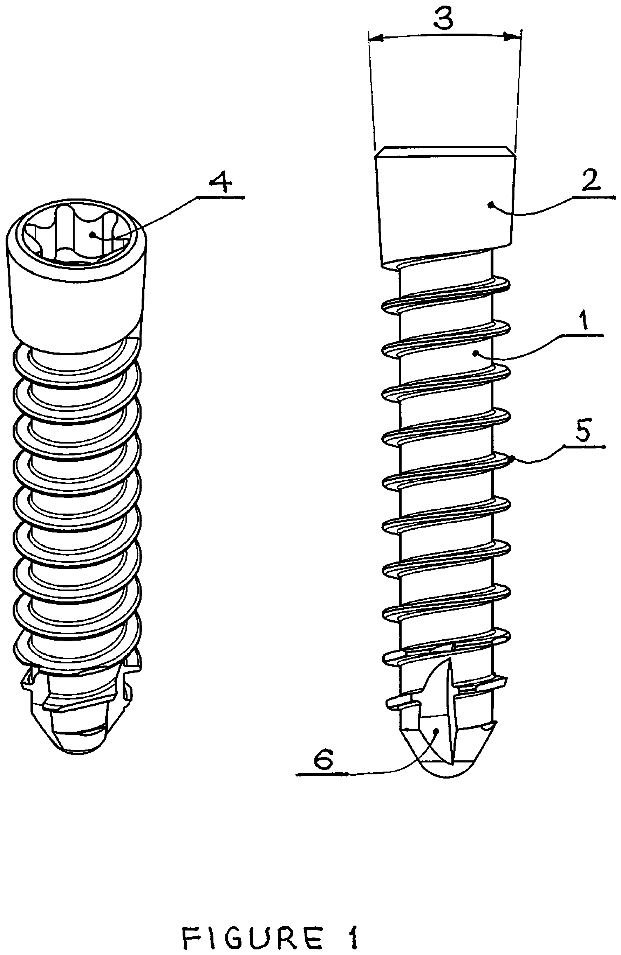

Figure 1 shows a bone screw 1 of the present invention with a conical head 2, tapered with a total cone angle 3. Angle 3 is smaller than required for the self-locking function of the Morse taper. The range of the angle 3 is usually limited on the lower end by the machining tolerances and is about 2.9 degrees corresponding to the taper of 1:20. On the upper end, an angle of 8 degrees would call for a high coefficient of friction not easily provided inside the body with lipids covering all implant surfaces. The compromise value of 5.7 degrees corresponding to the taper of 1:10, has been proven satisfactory. However, if the receiving holes in the plates are made with this angle, the compression between the head and the plate will be concentrated at the lower end of the screw head with a solid core and thus of higher stiffness. To reduce this stress concentration, the angle of the screw head should be larger than the angle of the receiving hole - a value of 6 degrees has been found to provide a satisfactory stress distribution with a screwdriver recess 4 of hexalobular type (Torx^{™}type). As the screw is inserted into the hole of the plate made with an angle of 5.7 degrees, the first contact occurs at the top of the hole and with the deformation of the head over the screwdriver recess with higher compliance, the contact widens to the bottom of the hole and the lower end of the screw head. This reduces the risk of cold welding. The threads 5 of the screw engage the bone following the threads cut in the bone by the cutting flutes 6 of the screw.

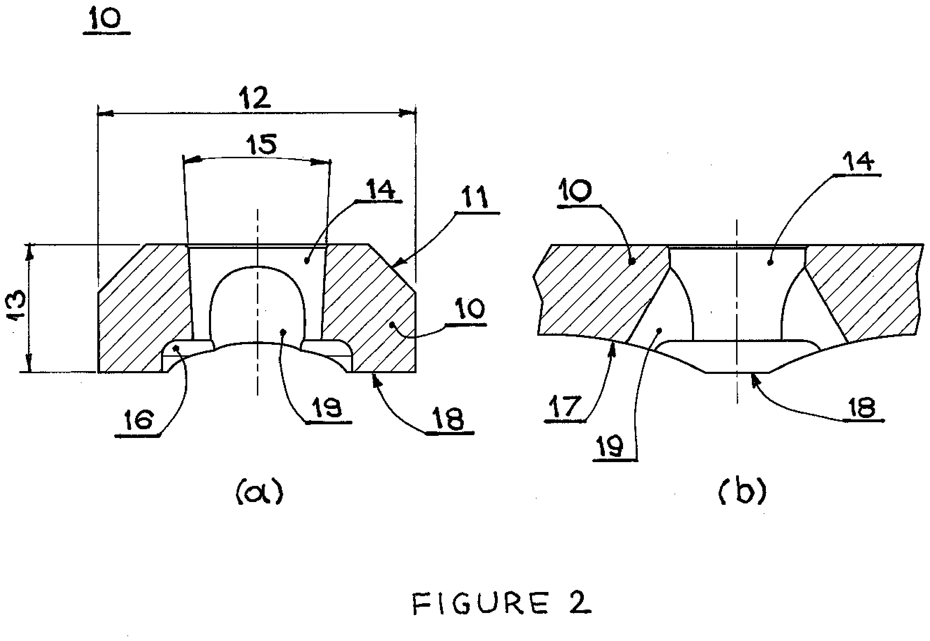

Figure 2 shows a transverse (a) and a longitudinal (b) cross-section of the bone plate 10 with a hole 14 for receiving the tapered head of the bone screw. The plate is of width 12 and height 13, with a facet 11 along the upper edge to facilitate soft tissue cover. The hole 14 is tapered towards the upper surface with a total angle 15 slightly smaller than the angle of the screw head. For the preferred self-locking combination with a nominal taper of 1:10, the angle of the hole is about 5.7 degrees and the head angle is about 6 degrees. On the bone-facing surface, the hole 14 is surrounded by a recess 16, which combined with a transverse cut 17 reduces the potential contact to the bone to small areas 18. On the lower side of the plate, the hole 14 is provided with longitudinally oriented cylindrical undercuts 19 which allow angulation of the screws.

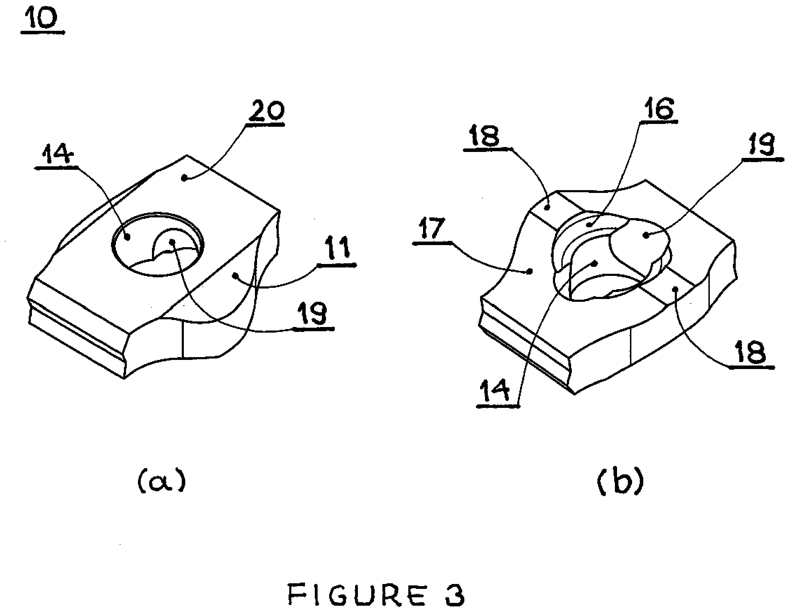

Figure 3 shows perspective views of a section of the plate 10 with a tapered hole 14. View (a) of the top 20 of the plate 10, shows the facet 11 and the undercut 19. View (b) of the bottom side of the plate shows the recess 16 surrounding the hole 14, the transverse cuts 17, the potential bone-contacting areas 18, and the cylindrical undercuts 19.

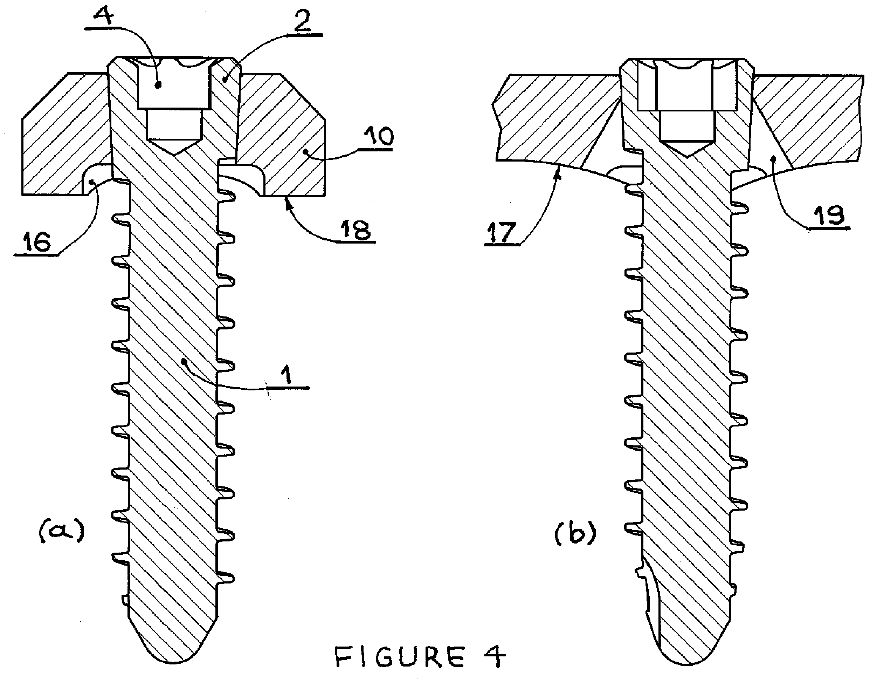

Figure 4 shows a transverse (a) and a longitudinal (b) cross-section of the bone plate 10 with a locking screw 1 inserted in the plate hole at 90 degrees. The head 2 of the screw 1 is fully seated and thus locked in the plate 10 in all degrees of freedom, forming a construct capable of transferring all loads between the plate and the bone. The plate and the screws become essentially a single implant unit. This is important not only for mechanical reasons for load transfer but also for avoidance of any movement between the plate and the screws that can lead to fretting corrosion and hence release of very fine metal particles and ions. Tissue response to such debris can have serious medical sequelae, locally but also systemically. Cylindrical undercuts 19 do reduce the contact area between the screw heads and the plate holes, but there is still sufficient contact on the sides of the holes to provide for safe locking.

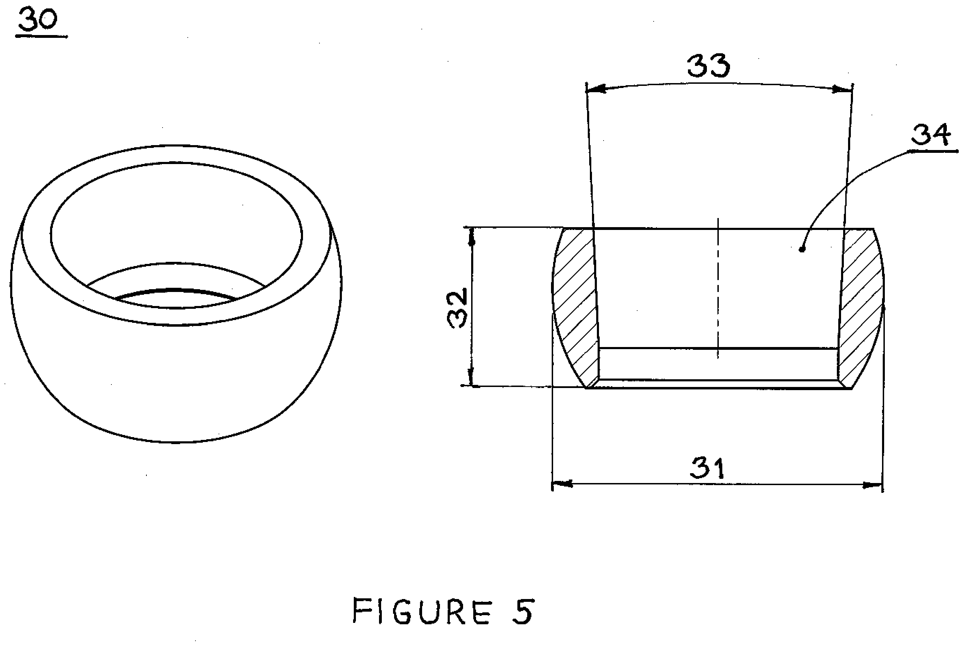

Figure 5 shows the bead head 30, the central item of this invention. The outside shape of the bead is a section of a sphere with the diameter 31. The height 32 of the bead is sufficient to cover most of the conical head of the screw inserted in the conical hole 34 with a total angle 33. For a nominal taper of 1:10, the angle 33 is about 5.7 degrees, i.e. about the same as the angle in the bone plate holes.

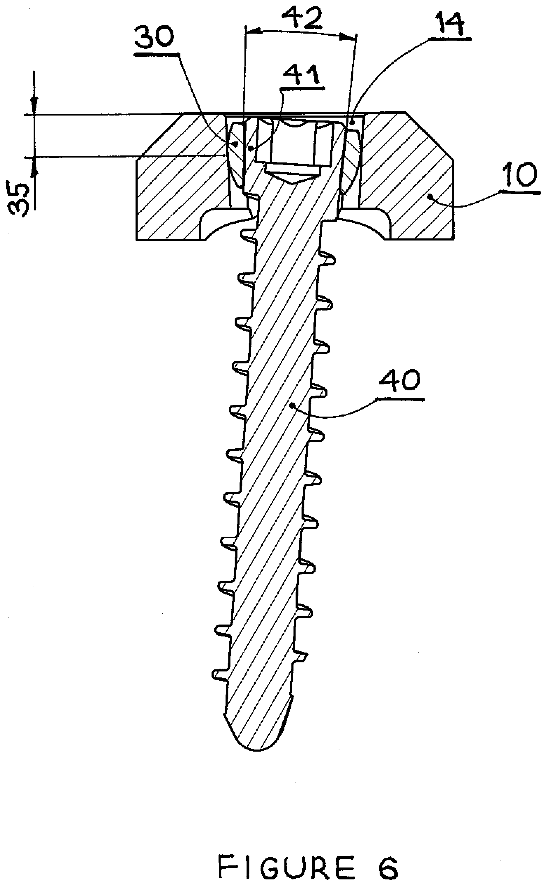

Figure 6 shows a transverse cross-section of the bone screw 40 inserted in the bone plate 10 through a bead 30. The diameter 31 of the bead is chosen and machined to a precise tolerance so that the depth 35 to which the bead is recessed in the hole 14 is equal to approximately a half, e.g. about 40% to about 60%, particularly about 50% of the hole height. In this cross-section the screw is slightly inclined with respect to the plate - the range usually called for is +- 5 degrees. The diameter of the screw 40 is smaller than the diameter of a nominal locking screw that fits in the hole 14 when inserted at 90 degrees.

For practical reasons of producing the beads and the selection of bone screws, the difference of the two screw diameters is about 0.9 mm to about 1.1 mm, particularly about 1 mm. The total angle 42 of the conical head 41 of the screw 40 is the same as the total angle 3 of the screw head 2 of the screw 1, Figure 1, i.e. preferably about 6 degrees.

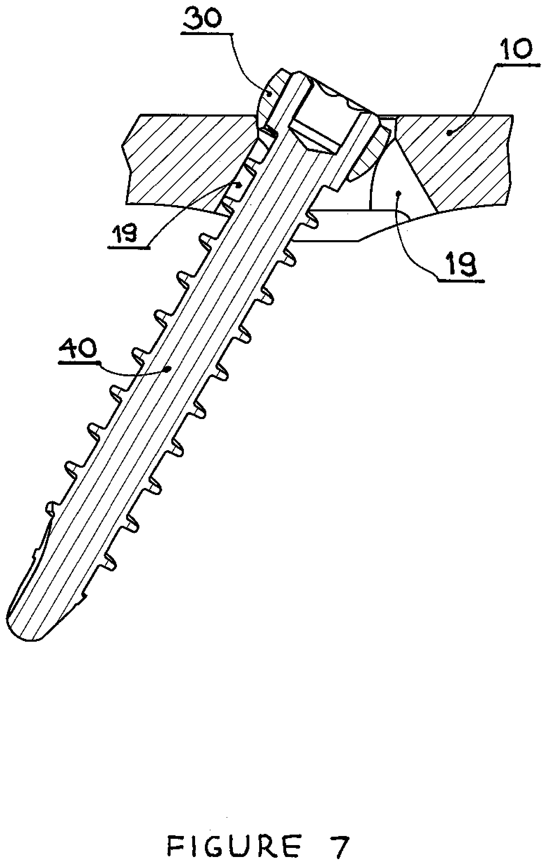

Figure 7 shows the screw 40 and the plate 10 in a longitudinal cross-section with the screw inclined to the maximum angle of about 30 degrees. This is made possible with the cylindrical undercuts 19 in the plate 10. The bead 30 is still engaging the conical hole of the plate 10 sufficiently to lock, but the contact is only a line contact and thus less resistant to bending loads. However, the screws are less likely to go loose from the bone by unscrewing and the potential for fretting is also substantially reduced compared to conventional screws inserted in conventional, non-locking plates.

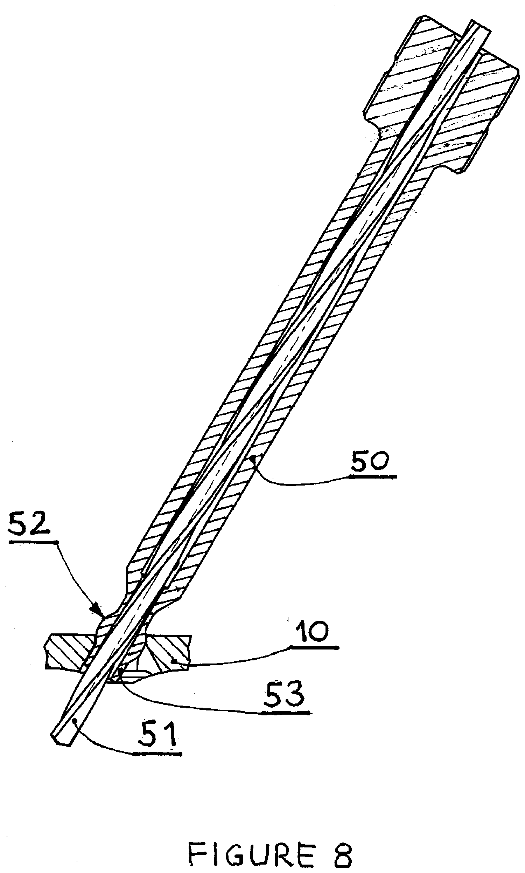

Figure 8 shows the drill sleeve 50 with a spherical tip 52 of the same diameter as the beads to be used in this hole of the plate 10. The sleeve is placed in the hole straight down and then inclined as needed. The nose 53 of the sleeve matches the outside diameter of the bone screw to be inserted at an angulation. The drill 51 matches the core diameter of the screw. Once a hole is drilled in the bone, the drill is retracted; the sleeve is turned to about 90 degrees to the plate and removed from the plate. Careful inspection of the geometry shows that the sleeve could not be removed from the plate hole while the drill is still in the bone without sliding the plate over the bone in a longitudinal direction.

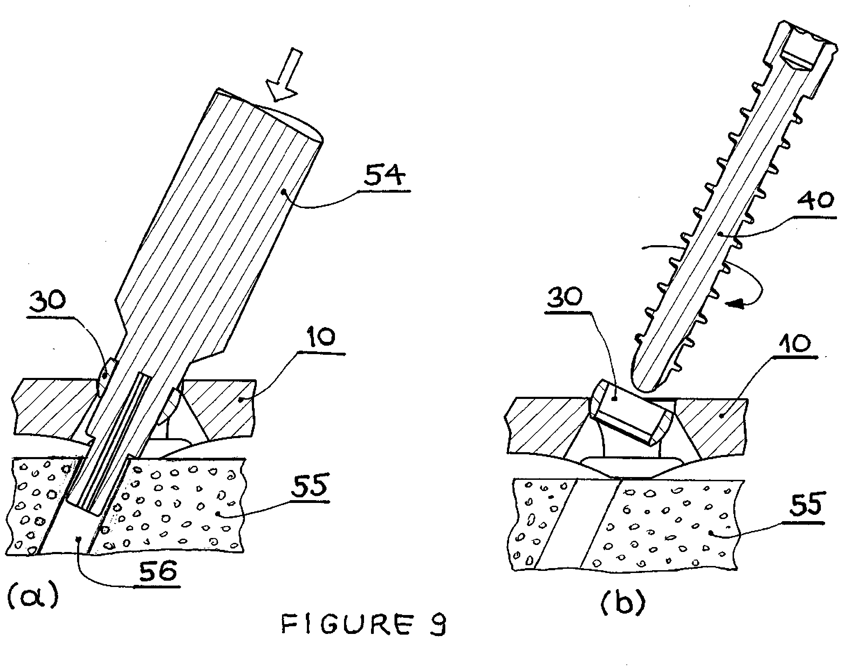

Figure 9 shows the sequence of insertion of the angulated screw. The bead 30 is held on a bead holder 54, cross-section (a) that can be inserted in the direction of the pre-drilled hole 56 in the bone 55. By a slight pressure on the bead 30 via the bead holder 54 the bead can be positioned in the plate hole where it will remain as the bead holder is removed. The bead holder is made from plastic and its tip is slotted, holding the bead just enough for handling.

Once the bead 30 is in place, cross-section (b), the screw 40 can be inserted through the bead and screwed into the bone 55, locking its head into the bead and the bead into the plate.

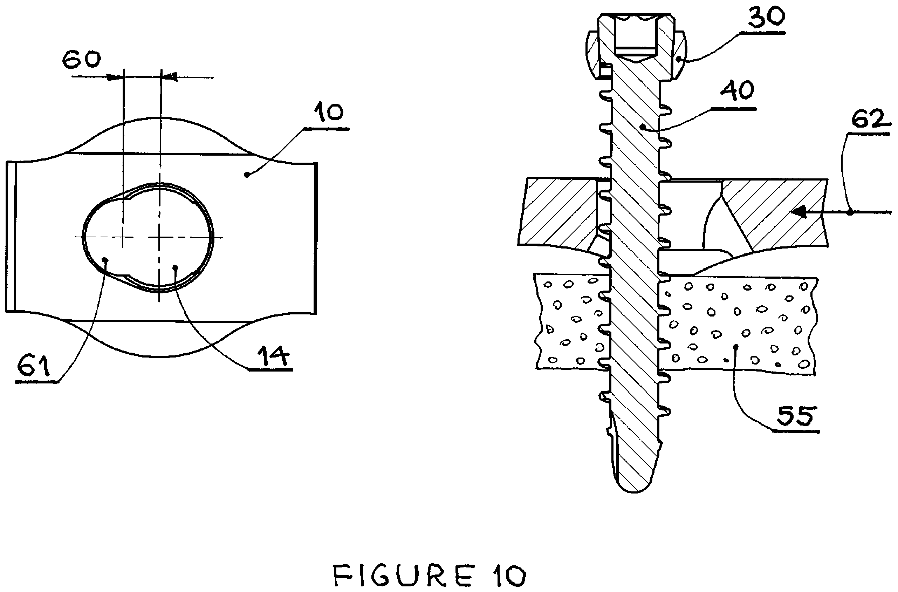

Figure 10 shows the use of a screw 40 augmented by the bead 30 for generating so-called dynamic compression. Some of the holes in the bone plate 10 are made as compression holes by extending the conical hole 14 into a hole 61, offset from the axis of the hole 14 by a distance 60. If the hole in the bone is drilled with a dedicated sleeve that can be centered in the hole 61, and the screw 40 with the bead 30 pre-placed on its head is inserted and screwed down into the bone 55 through the plate 10, the plate will be shifted longitudinally as shown by the arrow 62.

The invention of the screw head beads disclosed herein makes it possible to use, in addition to perpendicular, nominally sized locking screws with Morse taper heads, inclined screws of a smaller diameter in the bone plates with conical holes. It is also possible to use bead-augmented screws to create dynamic compression if needed. On a practical side, only one type screw - locking with a tapered head - can provide for all applications of bone plates. The use of beads in most cases is optional - only in peri-articular fractures and in rare cases where dynamic compression is possible and called for. An important advantage of using the beads, when indicated, is also the elimination of fretting between the screws and the plates, which is the main risk of detrimental tissue response to implants.

## Claims

1. An implant construct comprising a bone locking plate (10), a bone locking screw (40) and a spherical bead (30), wherein the bead (30) includes a conical hole (34) and is to be interposed between a conical head (41) of the bone locking screw (40) and a receiving hole (14) in the bone locking plate (10), **characterized in that** the receiving hole (14) in the bone locking plate (10) is conical.

2. The implant construct of claim 1 comprising a bone locking plate (10), a bone locking screw (40) and a spherical bead (30); wherein the bead (30) includes a conical hole (34) and is to be interposed between a conical head (41) of the bone locking screw (40) and a receiving hole (14) in the bone locking plate (10), **characterized in that** the receiving hole (14) in the bone locking plate (10) is conical, wherein the total angle (15) of the conical receiving hole (14) in the plate (10) is about the same as the total angle (33) of the conical hole (34) in the bead (30).

3. The implant construct of claim 1 or 2, wherein the total angle (42) of the conical screw head (41) is larger than the total angle (33) of the conical hole (34) in the bead (30) by about 0.2 to about 0.4 degrees preferably by about 0.3 degrees.

4. The implant construct of any one of claims 1-3, wherein the conical bead hole (34) is tapered with a self-locking angle (33).

5. The implant construction of any one of claims 1-4, wherein the conical bead hole angle (33) is between about 2.7 degrees and about 8 degrees, preferably about 5.7 degrees, corresponding to the taper of 1:10.

## Patentansprüche

1. Implantatkonstruktion umfassend eine Knochenverankerungsplatte (10), eine Knochenverankerungsschraube (40) und eine sphärische Kugel (30), wobei die Kugel (30) ein konisches Loch (34) umfasst und zwischen einem konischen Kopf (41) der Knochenverankerungsschraube (40) und einem Aufnahmeloch (14) in der Knochenverankerungsplatte (10) angeordnet werden soll, **dadurch gekennzeichnet, dass** das Aufnahmeloch (14) in der Knochenverankerungsplatte (10) konisch ist.

2. Implantatkonstruktion nach Anspruch 1 umfassend eine Knochenverankerungsplatte (10), eine Knochenverankerungsschraube (40) und eine sphärische Kugel (30); wobei die Kugel (30) ein konisches Loch (34) umfasst und zwischen einem konischen Kopf (41) der Knochenverankerungsschraube (40) und einem Aufnahmeloch (14) in der Knochenverankerungsplatte (10) angeordnet werden soll, **dadurch gekennzeichnet, dass** das Aufnahmeloch (14) in der Knochenverankerungsplatte (10) konisch ist, wobei der Gesamtwinkel (15) des konischen Aufnahmelochs (14) in der Platte (10) etwa der gleiche ist wie der Gesamtwinkel (33) des konischen Lochs (34) in der Kugel (30).

3. Implantatkonstruktion nach Anspruch 1 oder 2, wobei der Gesamtwinkel (42) des konischen Schraubenkopfes (41) um etwa 0,2 bis etwa 0,4 Grad größer ist als der Gesamtwinkel (33) des konischen Lochs (34) in der Kugel (30), vorzugsweise um etwa 0,3 Grad.

4. Implantatkonstruktion nach einem der Ansprüche 1 bis 3, wobei das konische Kugelloch (34) mit einem Selbsthemmungswinkel (33) verjüngt ist.

5. Implantatkonstruktion nach einem der Ansprüche 1 bis 4, wobei der Winkel des konischen Kugellochs (33) zwischen etwa 2,7 Grad und etwa 8 Grad, vorzugsweise etwa 5,7 Grad, beträgt, was einer Verjüngung von 1:10 entspricht.

## Revendications

1. Structure d'implant comprenant une plaque de blocage osseux (10), une vis de blocage osseux (40) et une bille sphérique (30), dans laquelle la bille (30) comprend un trou conique (34) et est conçue pour être interposée entre une tête conique (41) de la vis de blocage osseux (40) et un trou de réception (14) ménagé dans la plaque de blocage osseux (10), **caractérisée en ce que** le trou de réception (14) ménagé dans la plaque de blocage osseux (10) est conique.

2. Structure d'implant selon la revendication 1, comprenant une plaque de blocage osseux (10), une vis de blocage osseux (40) et une bille sphérique (30) ; dans laquelle la bille (30) comprend un trou conique (34) et est conçue pour être interposée entre une tête conique (41) de la vis de blocage osseux (40) et un trou de réception (14) ménagé dans la plaque de blocage osseux (10), **caractérisée en ce que** le trou de réception (14) ménagé dans la plaque de blocage osseux (10) est conique, dans laquelle l'angle total (15) du trou de réception conique (14) ménagé dans la plaque (10) est environ le même que l'angle total (33) du trou conique (34) ménagé dans la bille (30).

3. Structure d'implant selon la revendication 1 ou la revendication 2, dans laquelle l'angle total (42) de la tête de vis conique (41) est plus grand que l'angle total (33) du trou conique (34) ménagé dans la bille (30) d'environ 0,2 à environ 0,4 degré, de préférence d'environ 0,3 degré.

4. Structure d'implant selon l'une quelconque des revendications 1 à 3, dans laquelle le trou de bille conique (34) a une conicité ayant un angle d'autoblocage (33) .

5. Structure d'implant selon l'une quelconque des revendications 1 à 4, dans laquelle l'angle de trou de bille conique (33) est compris entre environ 2,7 degrés et environ 8 degrés, de préférence est égal à environ 5,7 degrés, correspondant à la conicité de 1:10.
